# EUROPEAN PATENT APPLICATION

(11) **EP 4 317 151 A1**
(43) Date of publication of application: **07.02.2024**
(21) Application number: 22799149.4
(22) Date of filing: 06.05.2022
(51) Int. Cl.: C07D 403/06, A61K 31/5377, A61P 3/04, A61P 3/10, A61P 29/00, A61P 15/10

(54) **CRYSTAL FORM IV OF ORGANIC ACID SALTS OF MELANOCORTIN RECEPTOR AGONIST COMPOUND, AND PREPARATION METHOD THEREOF**

(30) Priority: 07.05.2021 KR 20210059133
(71) Applicant: LG Chem, Ltd., Seoul 07336 (KR)
(72) Inventor: KIM, Ji Yoon, Daejeon 34122 (KR); CHUN, Seul Ah, Daejeon 34122 (KR); KIM, Sung Won, Daejeon 34122 (KR)
(74) Representative: Hoffmann Eitle
(86) International application number: PCT/KR2022/006480
(87) International publication number: WO 2022/235106

(57) **Abstract**

The present invention relates to a crystal form IV of organic acid salts of a compound represented by chemical formula 1, a preparation method thereof, and a pharmaceutical composition comprising same. The crystal form IV of organic acid salts of the compound represented by chemical formula 1 of the present invention may be characterized by an XRPD pattern, a DSC profile and/or a TGA profile.

## Description

### TECHNICAL FIELD

### [Cross-reference to Related Application]

The present invention claims the benefit of priority based on Korean Patent Application No. 10-2021-0059133, filed on May 7, 2021, the entire disclosure of which is incorporated as part of the specification.

### [Technical Field]

The present invention relates to a crystalline form IV of a novel compound exhibiting an excellent agonistic activity for a melanocortin receptor, a method for preparing the same, and a pharmaceutical composition comprising the same.

### BACKGROUND ART

Leptin protein is a hormone secreted by adipocytes, and its secretion amount increases with an increase in body fat content. It regulates functions of various neuropeptides produced from hypothalamus, thereby regulating various in vivo functions, including appetite, body fat content, and energy metabolism (Schwartz, et al., Nature 404, 661-671 (2000)). The leptin protein signal transduction for controlling appetite and body weight is made through the regulation of many factors downstream, the most representative of which are melanocortin, agouti-related peptide (AgRP), and neuropeptide Y (NPY) hormones.

When the concentration of leptin in the blood increases as a result of excess calories in vivo, the secretion of proopiomelanocortin (POMC) protein hormone from the pituitary gland increases and the production of AgRP and NPY decreases. A small peptide hormone, alpha-melanocyte-stimulating hormone (MSH), is produced from POMC neurons. The hormone is an agonist for melanocortin-4 receptors (MC4R) of second-order neurons and ultimately induces appetite decrease. Meanwhile, when the concentration of leptin decreases as a result of calorie deficiency, the expression of AgRP, an MC4R antagonist, increases, and the expression of NPY also increases, which ultimately promotes appetite. That is, according to the change of leptin, the alpha-MSH hormone and the AgRP hormone act as agonists and antagonists for MC4R and thus are involved in appetite control.

The Alpha-MSH hormone binds to three MCR subtypes in addition to MC4R to induce various physiological reactions. Five MCR subtypes have been identified so far. Among them, MC1R is expressed mainly in skin cells and is involved in regulating melanin pigmentation (skin pigmentation). MC2R is expressed mainly in the adrenal gland and is known to be involved in the production of glucocorticoid hormones. Its ligand is only adrenocorticotropic hormone (ACTH) derived from POMC. MC3R and MC4R, which are expressed mainly in the central nervous system, are involved in regulating appetite, energy metabolism, and body fat storage efficiency, and MC5R expressed in various tissues is known to regulate exocrine function (Wikberg, et al., Pharm Res 42 (5) 393-420 (2000)). In particular, activation of the MC4R receptor induce appetite decrease and energy metabolism increase and thus has an effect of efficiently reducing body weight. Therefore, it has been proven to be a major action point in the development of anti-obesity drugs (Review: Wikberg, Eur. J. Pharmacol 375, 295-310 (1999)); Wikberg, et al., Pharm Res 42 (5) 393-420 (2000); Douglas et al., Eur J Pharm 450, 93-109 (2002); O'Rahilly et al., Nature Med 10, 351-352 (2004)).

The role of MC4R in the control of appetite and body weight was primarily demonstrated through an experiment in an animal model of abnormal expression of the agouti protein (agouti mouse). In the case of the Agouti mouse, it was found that due to genetic mutation, the agouti protein was expressed at a high concentration in the central nervous system and acted as an antagonist of MC4R in the hypothalamus to cause obesity (Yen, TT et al., FASEB J. 8, 479-488 (1994); Lu D., et al. Nature 371, 799-802 (1994)). Subsequent research results showed that the agouti-related peptides (AgRP) similar to the actual agouti protein were expressed in hypothalamic nerves, and these are also known to be antagonists for MC4R and be involved in controlling appetite (Shutter, et al., Genes Dev., 11, 593-602 (1997); Ollman, et al. Science 278, 135-138 (1997)).

Intracerebral administration of alpha-MSH, which is an in vivo MC4R agonist, to animals leads to the effect of reducing appetite. When treating the animals with SHU9119 (peptide) or HS014 (peptide), which are MC4R antagonists, it was observed that appetite increased again (Kask et al., Biochem. Biophys. Res. Comm. 245, 90-93 (1998)). In addition, in animal studies using Melanotan II (MTII, Ac-Nle-c[Asp-His-DPhe-Arg-Trp-Lys]-NH2) and the similar agonist thereof, HP228, after intracerebral, intraperitoneal, or subcutaneous administration, efficiencies of inhibiting appetite, reducing body weight, increasing energy metabolism, etc. were found. (Thiele T. E., et al. Am J Physiol 274 (1 Pt 2), R248-54 (1998); Lee M. D., et al. FASEB J 12, A552 (1998); Murphy B., et al. J Appl Physiol 89, 273-82 (2000)). On the contrary, administration of the representative SHU9119 to animals showed significant and sustained feed intake and weight gain, providing pharmacological evidence that MCR agonists could be anti-obesity agents. The effect of reducing appetite, which is clearly exhibited upon administration of MTII, was not observed in MC4R knock-out (KO) mice. This experimental result proves again that the appetite-reducing effect is achieved mainly through the activation of MC4R (Marsh, et al., Nat Genet 21, 119-122 (1999)).

Anorectic agents acting on the central nervous system were the main types of anti-obesity drugs developed so far. Among them, most were drugs that modulate the action of neurotransmitters. Examples include noradrenalin agents (phentermine and mazindol), serotonergic agents, fluoxetine and sibutramine, and the like. However, the neurotransmitter modulators have a wide range of effects on various physiological actions in addition to appetite suppression, through numerous subtype receptors. Accordingly, the modulators lack selectivity for each subtype, and thus have a major disadvantage in that they are accompanied by various side effects when administered for a long period.

Meanwhile, melanocortin agonists are neuropeptides, not neurotransmitters. Given that in MC4R gene KO mice, all functions other than energy metabolism are normal, they have an advantage as an action point in that they can induce only weight loss through appetite suppression without affecting other physiological functions. In particular, the receptor is a G-protein coupled receptor (GPCR) that belongs to the most successful category of new drug action points developed so far. Thus, the action point greatly differ from existing action points in that it is relatively easy to secure selectivity for subtype receptors.

As an example of utilizing a melanocortin receptor as an action point, international publication nos. WO 2008/007930 and WO 2010/056022 disclose compounds as agonists of the melanocortin receptor.

In addition, the inventors of the present invention have conducted extensive studies and invented a novel compound of the following formula 1 having an excellent agonistic activity selective for a melanocortin receptor, in particular, melanocortin-4 receptor (MC4R), and a method for preparing the same (application no. KR 10-2019-0141649 (filed on 7 November 2019)): 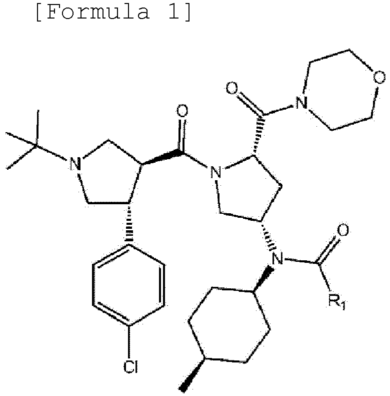 (wherein R₁ is a C₂-C₅ alkyl.) .

Meanwhile, the crystal structure of a pharmaceutically active ingredient often affects the chemical stability of the drug. Different crystallization conditions and storage conditions can lead to changes in the crystal structure of the compound, and sometimes the accompanying production of other forms of the crystalline form. In general, an amorphous drug product does not have a regular crystal structure, and often has other defects such as poor product stability, smaller particle size, difficult filtration, easy agglomeration, and poor flowability. Thus, it is necessary to improve various physical properties of the product. As such, it is necessary to study crystal structures having high purity and good chemical stability for a single compound.

### [Prior art documents]

### [Patent Documents]

(Patent Document 1) International Patent Application Publication No. WO 2008/007930
(Patent Document 2) International Patent Application Publication No. WO 2010/056022

### DISCLOSURE OF THE INVENTION

### TECHNICAL PROBLEM

An aspect of the present invention provides a stable crystalline form of a novel compound having an excellent agonistic activity, which is selective for a melanocortin receptor, in particular, melanocortin-4 receptor (MC4R), and a method for preparing the same.

Another aspect of the present invention provides a pharmaceutical composition comprising the stable crystalline form of the novel compound.

### TECHNICAL SOLUTION

According to an aspect of the present invention,
there is provided a crystalline form IV of an organic acid salt of a compound of the following formula 1, wherein the X-ray powder diffraction (XRPD) pattern has characteristic peaks with the diffraction angles (2θ values) described in i), ii), or iii):
i) 5 or more characteristic peaks selected from among 7.6310.2°, 9.3610.2°, 9.6010.2°, 12.59±0.2°, 14.0±0.2°, 14.99±0.2°, 15.2610.2°, 16.7610.2°, 17.8±0.2°, 18.8610.2°, 18.8910.2°, 19.00±0.2°, 19.21±0.2°, 19.37±0.2°, 20.13±0.2°, 20.38±0.2°, 20.6310.2°, 20.72±0.2°, 22.14±0.2°, and 22.3310.2°;
ii) 5 or more characteristic peaks selected from among 8.47±0.2°, 10.8610.2°, 11.99±0.2°, 12.21±0.2°, 13.47±0.2°, 15.08±0.2°, 15.19±0.2°, 16.80±0.2°, 17.25±0.2°, 17.45±0.2°, 17.8610.2°, 18.77±0.2°, 20.2610.2°, 20.44±0.2°, 21.51±0.2°, 21.81±0.2°, 22.00±0.2°, 22.6610.2°, 24.97±0.2°, and 25.3610.2°;
iii) 5 or more characteristic peaks selected from among 4.27±0.2°, 6.5610.2°, 8.87±0.2°, 9.8610.2°, 14.7210.2°, 15.1610.2°, 15.74±0.2°, 16.1410.2°, 16.7210.2°, 16.9110.2°, 17.09±0.2°, 17.27±0.2°, 17.52±0.2°, 17.81±0.2°, 18.0010.2°, 18.3310.2°, 19.6710.2°, 20.30±0.2°, 21.23±0.2°, and 23.97±0.2°, 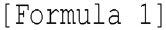 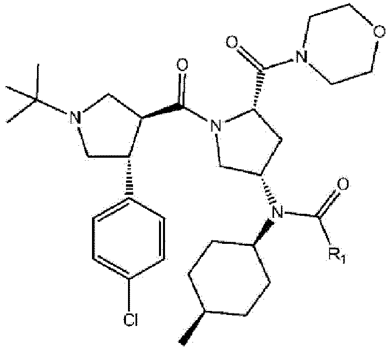 wherein
   R₁ is a C₂-C₅ alkyl.

Since the compound of formula 1 can have an asymmetric carbon center and an asymmetric axis or an asymmetric plane, it can exist as cis or trans isomers, R or S isomers, racemates, diastereomer mixtures, and individual diastereomers, all of which are within the scope of the compound of formula 1.

In the present specification, unless otherwise specified for convenience, the compound of formula 1 is used to include all of the compound of formula 1, an isomer thereof, and a solvate thereof.

In one embodiment, according to the present invention, in formula 1, R₁ is a C₂ to C₅ alkyl. In another embodiment, according to the present invention, in formula 1, R₁ is a straight-chain or branched C₂ to C₅ alkyl, for example, ethyl, n-propyl, iso-propyl, n-butyl, iso-butyl, sec-butyl, or tert-butyl.

In another embodiment, according to the present invention, in formula 1, R₁ is a C₂ to C₄ alkyl. In another embodiment, according to the present invention, in formula 1, R₁ is a straight-chain or branched C₂ to C₄ alkyl, for example, ethyl, n-propyl, iso-propyl, n-butyl, sec-butyl, or tert-butyl. Specifically, R₁ may be iso-propyl.

In one embodiment according to the present invention, the solvate may include a hydrate; and a solvate with an organic solvent, such as methanol, ethanol, 2-propanol, 1,2-propanediol, 1,3-propanediol, n-butanol, 1,4-butanediol, tertbutanol, acetic acid, acetone, butyl acetate, methyl acetate, ethyl acetate, propyl acetate, t-butyl acetate, isobutyl acetate, methylethylketone, 2-pentanone, tetrahydrofuran, acetonitrile, chloroform, toluene, and mixtures thereof.

The present invention provides a crystalline form IV of an organic acid salt of the compound of formula 1.

In one embodiment, according to the present invention, the organic acid salt is a pharmaceutically acceptable salt, and may include, but is not limited to, acid-addition salts which are formed from inorganic acids, such as hydrochloric acid, sulfuric acid, nitric acid, phosphoric acid, hydrobromic acid, and hydroiodic acid; organic carboxylic acids, such as tartaric acid, formic acid, citric acid, acetic acid, trichloroacetic acid, trifluoroacetic acid, gluconic acid, benzoic acid, lactic acid, fumaric acid, and maleic acid; or sulfonic acids, such as methanesulfonic acid, benzenesulfonic acid, p-toluenesulfonic acid, or naphthalene-sulfonic acid.

In another embodiment, according to the present invention, the organic acid salt of the compound of formula 1 may be an acid addition salt formed from at least one organic acid selected from the group consisting of lactic acid, benzene sulfonic acid, and toluene sulfonic acid.

In another embodiment, according to the present invention, the organic acid salt selected from the group consisting of lactate, benzene sulfonate, and toluene sulfonate of the compound of formula 1 may have superior physical properties, such as stability and solubility of the compound, to inorganic acid salts or organic acid salts formed from organic acids, other than lactic acid, benzene sulfonic acid, and toluene sulfonic acid, of the compound of formula 1. However, the effects of the present invention are not limited thereto.

In one embodiment, according to the present invention, the crystalline form IV of the organic acid salt of the compound of formula 1 may be crystalline form A of lactate of the compound of formula 1 having 5 or more, 7 or more, 9 or more, 10 or more, 13 or more, 15 or more, 17 or more, or 20 or more characteristic peaks selected from among 7.6310.2°, 9.3610.2°, 9.6010.2°, 12.59±0.2°, 14.0±0.2°, 14.99±0.2°, 15.2610.2°, 16.7610.2°, 17.8±0.2°, 18.8610.2°, 18.8910.2°, 19.00±0.2°, 19.21±0.2°, 19.37±0.2°, 20.13±0.2°, 20.38±0.2°, 20.6310.2°, 20.72±0.2°, 22.14±0.2°, and 22.33±0.2° in the X-ray powder diffraction pattern.

The crystalline form A, according to one embodiment, according to the present invention, may have characteristic peaks of 7.6310.2°, 9.3610.2°, 9.6010.2°, 12.59±0.2°, 14.0±0.2°, 14.99±0.2°, 15.2610.2°, 16.7610.2°, 17.8±0.2°, 18.8610.2°, 18.8910.2°, 19.00±0.2°, 19.21±0.2°, 19.37±0.2°, 20.13±0.2°, 20.38±0.2°, 20.6310.2°, 20.72±0.2°, 22.14±0.2°, and 22.33±0.2° in the X-ray powder diffraction pattern.

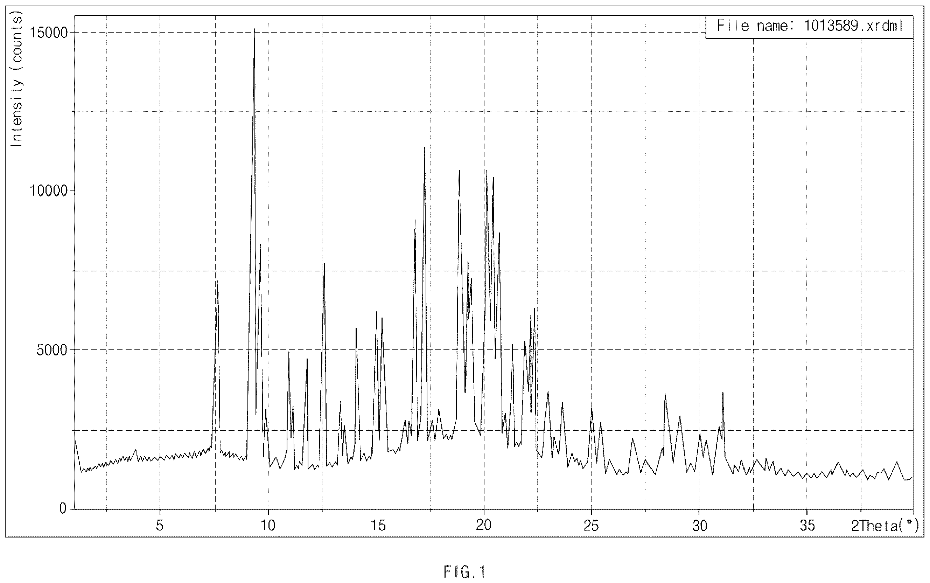

In one embodiment, according to the present invention, the crystalline form A may have the X-ray powder diffraction (XRPD) pattern shown in FIG. 1.

In one embodiment, according to the present invention, the crystalline form A, in the thermogravimetric analysis (TGA) profile, may have 5% or less, 4% or less, 3% or less, 2% or less, 1% or less, or 1% of a weight loss in a temperature range of about 38°C to 110°C, and/or may be decomposed beyond about 274°C.

In one embodiment, according to the present invention, the crystalline form A, in the differential scanning calorimetry (DSC) profile, may have an endothermic peak at about 80°C to 110°C, which is within a temperature range at which a weight loss occurs in the TGA profile, and may have an exothermic peak at about 110°C to 120°C.

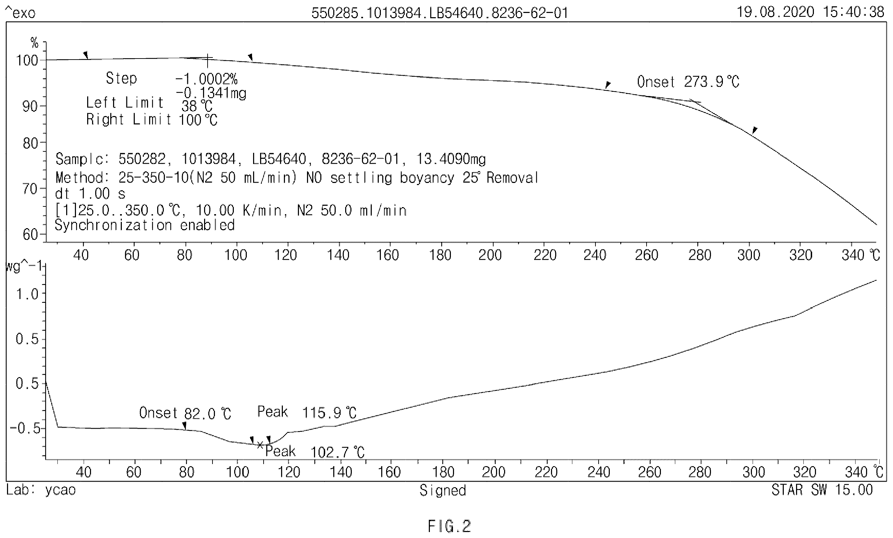

In one embodiment, according to the present invention, the crystalline form A may have the TGA (top) and/or DSC (bottom) profiles shown in FIG. 2.

In one embodiment, according to the present invention, the crystalline form A may be melted at about 84.2°C to 112.7°C, as determined by hot stage microscopy (HSM).

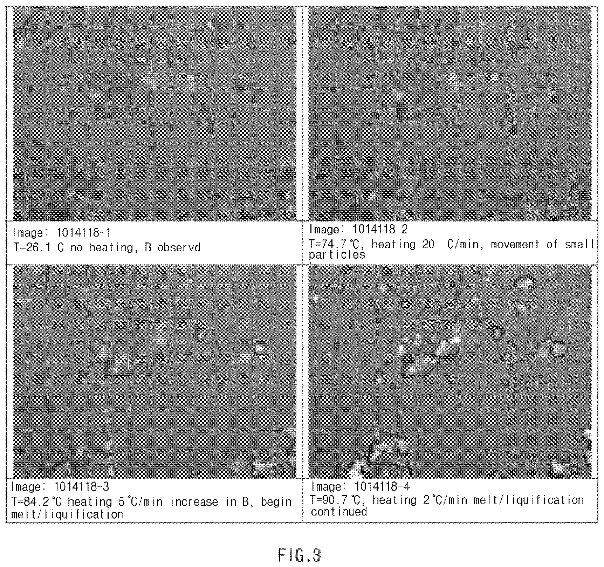

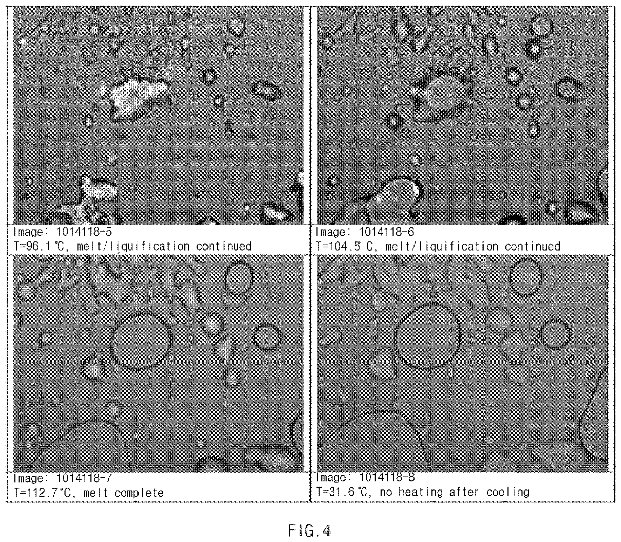

In one embodiment, according to the present invention, the crystalline form A may have the HSM photographs shown in FIGs. 3 and 4.

In one embodiment, according to the present invention, the crystalline form A may have a solubility of more than 24 mg/mL in an aqueous solution.

In another embodiment, according to the present invention, the crystalline form IV of the organic acid salt of the compound of formula 1 may be crystalline form B of benzene sulfonate of the compound of formula 1 having 5 or more, 7 or more, 9 or more, 10 or more, 13 or more, 15 or more, 17 or more, or 20 or more characteristic peaks selected from among 8.47±0.2°, 10.8610.2°, 11.99±0.2°, 12.21±0.2°, 13.47±0.2°, 15.08±0.2°, 15.19±0.2°, 16.80±0.2°, 17.25±0.2°, 17.45±0.2°, 17.8610.2°, 18.77±0.2°, 20.2610.2°, 20.44±0.2°, 21.51±0.2°, 21.81±0.2°, 22.00±0.2°, 22.6610.2°, 24.97±0.2°, and 25.36±0.2° in the X-ray powder diffraction pattern.

The crystalline form B, according to another embodiment, according to the present invention, may have characteristic peaks of 8.47±0.2°, 10.8610.2°, 11.99±0.2°, 12.21±0.2°, 13.47±0.2°, 15.08±0.2°, 15.19±0.2°, 16.80±0.2°, 17.25±0.2°, 17.45±0.2°, 17.86±0.2°, 18.77±0.2°, 20.26±0.2°, 20.44±0.2°, 21.51±0.2°, 21.81±0.2°, 22.00±0.2°, 22.66±0.2°, 24.97±0.2°, and 25.36±0.2° in the X-ray powder diffraction pattern.

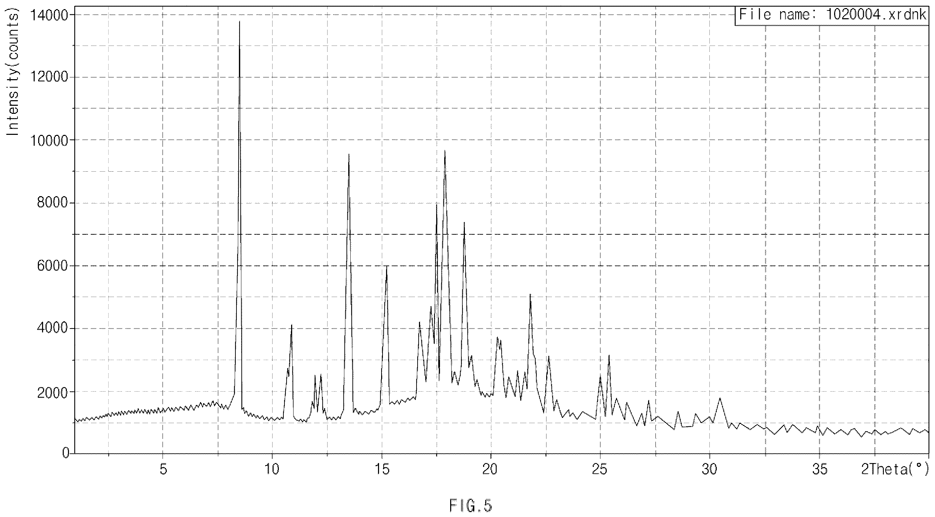

In another embodiment, according to the present invention, the crystalline form B may have the X-ray powder diffraction (XRPD) pattern shown in FIG. 5.

In another embodiment, according to the present invention, the crystalline form B, in the thermogravimetric analysis (TGA) profile, may have 10% or less, 8% or less, 6% or less, 5.5% or less, or 5.4% of a weight loss in a temperature range of about 39°C to 150°C, and/or may be decomposed beyond about 266°C. The weight loss may indicate that 2.5 moles of water (H₂O) evaporates in the temperature range, but the feature of the present invention is not limited thereto.

In another embodiment, according to the present invention, the crystalline form B, in the differential scanning calorimetry (DSC) profile, may have a broad endothermic peak at about 57°C to 93°C, which is within a temperature range at which a weight loss occurs in the TGA profile.

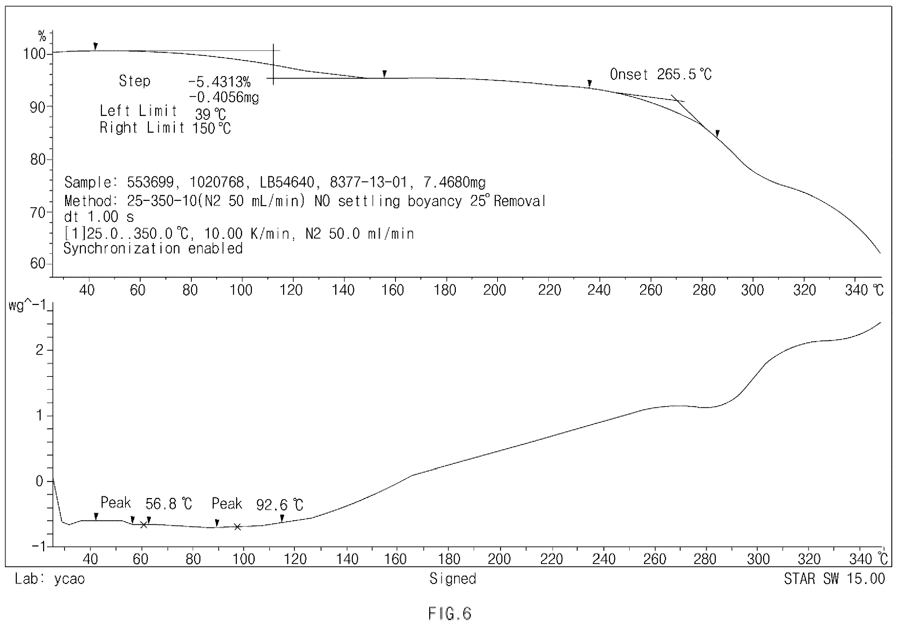

In another embodiment, according to the present invention, the crystalline form B may have the TGA (top) and/or DSC (bottom) profiles shown in FIG. 6.

In another embodiment, according to the present invention, the crystalline form B may be melted at about 122°C~ 157°C, specifically, 121.8°C to 157.8°C, as determined by hot stage microscopy (HSM).

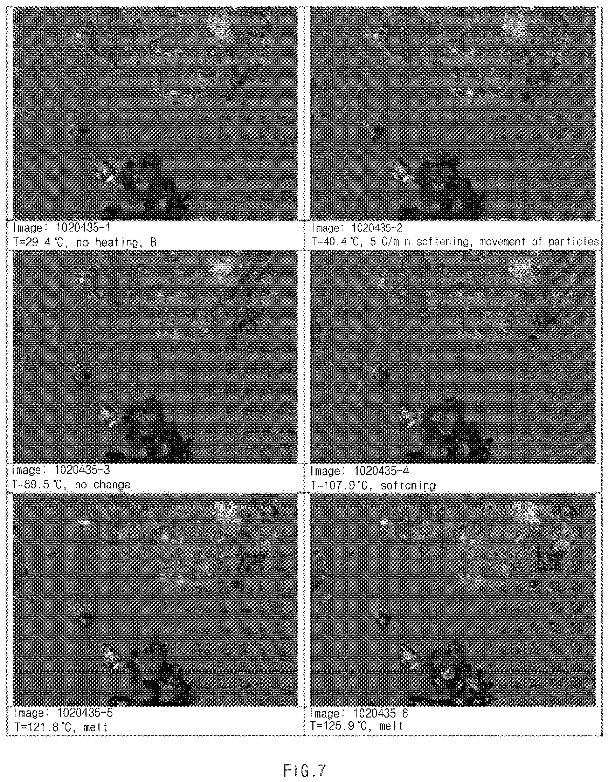

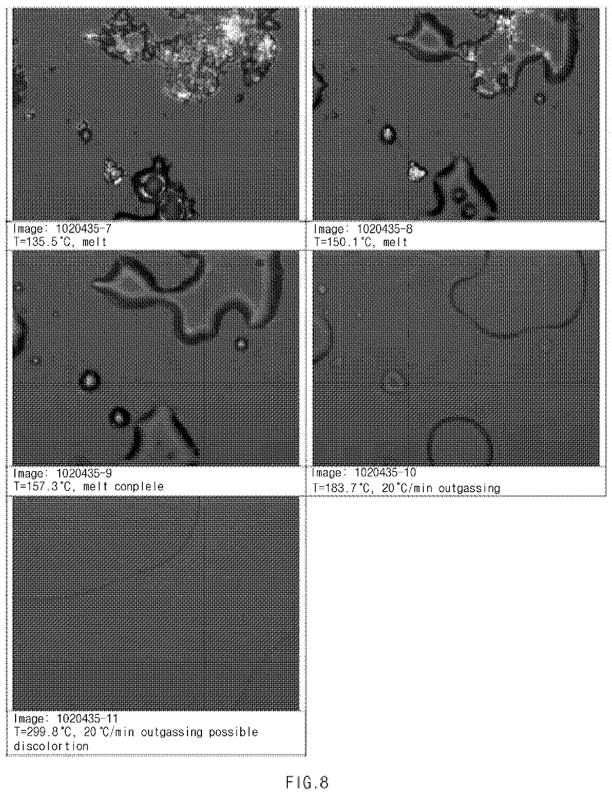

In another embodiment, according to the present invention, the crystalline form B may have the HSM photographs shown in FIGs. 7 and 8.

In another embodiment, according to the present invention, the crystalline form B may have a solubility of more than 1 mg/mL in an aqueous solution.

In another embodiment, according to the present invention, the crystalline form IV of the organic acid salt of the compound of formula 1 may be crystalline form C of toluene sulfonate of the compound of formula 1 having 5 or more, 7 or more, 9 or more, 10 or more, 13 or more, 15 or more, 17 or more, or 20 or more characteristic peaks selected from among 4.27±0.2°, 6.5610.2°, 8.87±0.2°, 9.8610.2°, 14.7210.2°, 15.1610.2°, 15.74±0.2°, 16.1410.2°, 16.7210.2°, 16.9110.2°, 17.09±0.2°, 17.27±0.2°, 17.52±0.2°, 17.81±0.2°, 18.0010.2°, 18.3310.2°, 19.6710.2°, 20.30±0.2°, 21.23±0.2°, and 23.97±0.2° in the X-ray powder diffraction pattern.

The crystalline form C, according to another embodiment, according to the present invention, may have characteristic peaks of 4.27±0.2°, 6.5610.2°, 8.87±0.2°, 9.8610.2°, 14.72±0.2°, 15.1610.2°, 15.74±0.2°, 16.1410.2°, 16.7210.2°, 16.9110.2°, 17.09±0.2°, 17.27±0.2°, 17.52±0.2°, 17.81±0.2°, 18.00±0.2°, 18.33±0.2°, 19.67±0.2°, 20.30±0.2°, 21.23±0.2°, and 23.97±0.2° in the X-ray powder diffraction pattern.

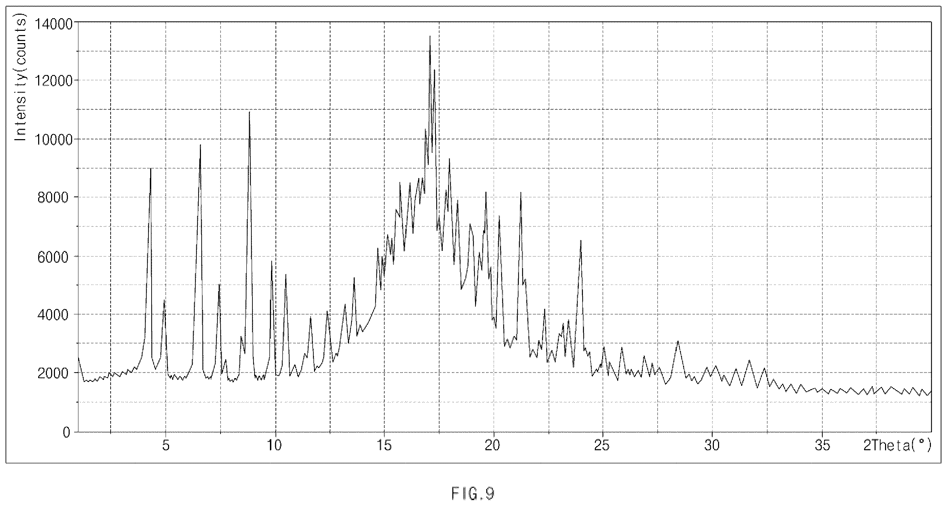

In another embodiment, according to the present invention, the crystalline form C may have the X-ray powder diffraction (XRPD) pattern shown in FIG. 9.

In another embodiment, according to the present invention, the crystalline form C, in the thermogravimetric analysis (TGA) profile, may have 10% or less, 8% or less, 6% or less, 5.5% or less, or 5.3% of a weight loss in a temperature range of about 46°C to 135°C, and/or may be decomposed beyond about 264°C.

In another embodiment, according to the present invention, the crystalline form C, in the differential scanning calorimetry (DSC) profile, may have a broad endothermic peak at about 104°C, which is within a temperature range at which a weight loss occurs in the TGA profile. In addition, the crystalline form C may have an exothermic peak at about 138°C. The broad endothermic peak may be due to loss of a volatile substance, but the feature of the present invention is not limited thereto.

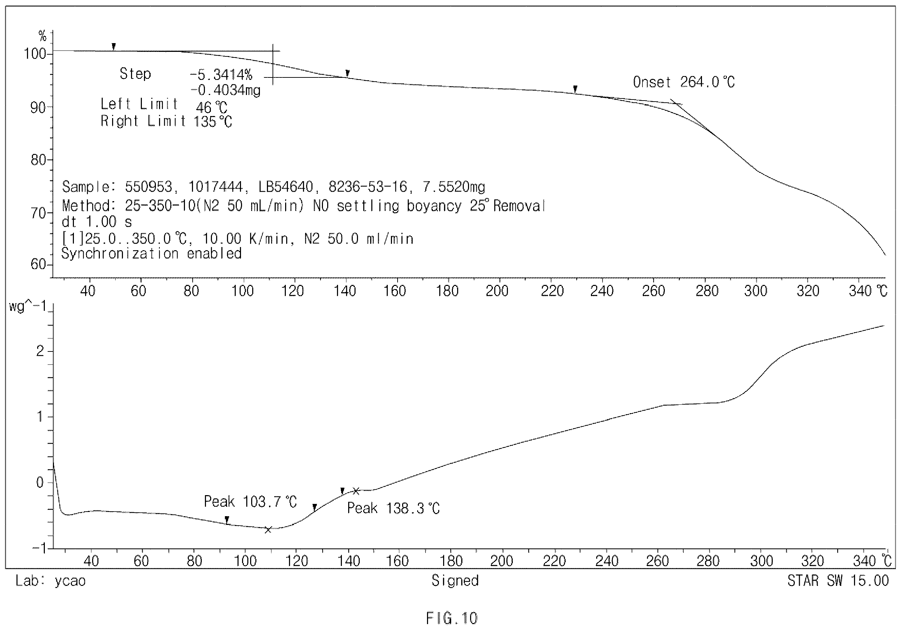

In another embodiment, according to the present invention, the crystalline form C may have the TGA (top) and/or DSC (bottom) profiles shown in FIG. 10.

In another embodiment, according to the present invention, the crystalline form C may be a crystalline form of a solvate with methyl tertiary-butyl ether (MTBE, methyl tert-butyl ether) of toluene sulfonate of the compound of formula 1. This may be confirmed by the collapse of the crystal structure during desolvation of the crystalline form IV, according to another embodiment of the present invention.

In another embodiment, according to the present invention, according to the ¹H NMR result spectrum, the crystalline form C may have the same structure as the compound of formula 1, and a peak shift may be observed to indicate that the salt is formed.

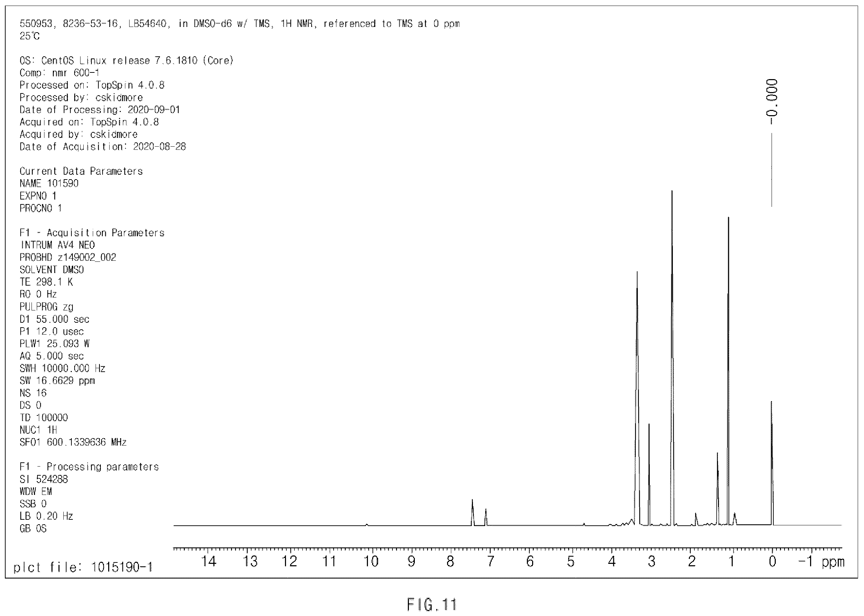

In another embodiment, according to the present invention, the crystalline form C may have the 1H NMR spectrum shown in FIG. 11. Herein, the crystalline form C of toluene sulfonate may contain the parent compound and toluene sulfonic acid in a molar ratio of 1:1 and may contain MTBE in a molar ratio of 0.8. Herein, it was confirmed that the crystalline form C of another embodiment according to the present invention may be a crystalline form of a solvate by the collapse of the crystal structure during desolvation of the crystalline form C, but the present invention is not limited thereto.

In another embodiment, according to the present invention, the crystalline form C may have a solubility of more than 10 mg/mL in an aqueous solution.

In the present specification,
X-ray powder diffraction (XRPD) analysis shows the results obtained with PANalytical X' Pert Pro MPD system (Malvern Panalytical Ltd.).

Thermogravimetric analysis (TGA) and differential scanning calorimetry (DSC) analysis show the results obtained with TGA/DSC 3 (Mettler-Toledo AG).

Hot stage microscopy (HSM) shows the results obtained with Leica DM LP microscope using Linkman hot stage (model FTIR 600) with TMS 93 controller.

Solubility indicates the result of measurement based on the final volume of water added to a sample when it is visually confirmed that the sample is completely dissolved by adding water to the sample. The solubility of the actual sample may be higher than the measured solubility.

¹H NMR shows the result of measurement using Bruker Advance 600 MHz NMR spectrometer.

Herein, the temperature values may have an error of ±5°C.

The crystalline form IV may have higher purity than a crude compound of formula 1, an amorphous compound of formula 1, other pharmaceutically acceptable salts of the compound of formula 1, or other crystalline forms of the compound of formula 1, and may be physically and chemically more stable.

In addition, the agonistic ability for the melanocortin-4 receptor and preventive or therapeutic effects on diseases, such as obesity, diabetes, inflammation, erectile dysfunction, or the like, of the crystalline form IV of the organic acid salt of the compound of formula 1, may be more excellent than those of known melanocortin-4 receptor agonists. However, the effects of the present invention are not limited thereto.

In another aspect, the present invention provides a method for preparing the crystalline form IV, comprising the steps of: preparing a mixed solution in which the compound of formula 1 and an organic acid are mixed in a first crystallization solvent; and obtaining crystals from the mixed solution.

First, the compound represented by formula 1 and an organic acid are dissolved in a crystallization solvent (which may also be referred to as a 'first crystallization solvent' to distinguish from a crystallization solvent ('second crystallization solvent') that is additionally introduced in any additional step in the future).

The compound of formula 1 for preparing the crystalline form IV may be a compound of formula 1, a salt thereof, an isomer thereof, or a solvate thereof.

The compound of formula 1 may be obtained by the preparation method described in the specification of application no. KR 10-2019-0141649 (filed on 7 November 2019) .

The organic acid may be used without particular limitation as long as it can form a pharmaceutically acceptable acid addition salt of the compound of formula 1.

In one embodiment, according to the present invention, the organic acid may be at least one selected from the group consisting of lactic acid, benzene sulfonic acid, and toluene sulfonic acid.

The first crystallization solvent may be used without particular limitation as long as it is a suitable solvent for crystallization of compounds. In one embodiment, the crystalline solvent may include an organic solvent.

The organic solvent may include aliphatic linear saturated hydrocarbon-based solvents, such as pentane, hexane, and heptane; alicyclic hydrocarbon-based solvents, such as cyclopentane, cyclohexane, and cycloheptane; dialkyl ether-based solvents, such as diethyl ether, dipropyl ether, dibutyl ether, diisoamyl ether, ethyl methyl ether, methyl propyl ether, methyl butyl ether, and ethyl propyl ether; cyclic ether-based solvents, such as tetrahydrofuran and tetrahydropyran; aromatic ring-containing ether-based solvents, such as, diphenyl ether and anisole; amide-based solvents, such as dimethylacetamide; ketone-based solvents, such as methyl isobutyl ketone; ester-based solvents, such as isopropyl acetate and isobutyl acetate; alcohol-based solvents, such as ethanol, propyl alcohol, butanol, and pentanol; toluene-based solvents, such as toluene; or mixtures thereof.

In another embodiment, according to the present invention, the first crystallization solvent may include ethanol, toluene, methyl tertiary-butyl ether (methyl tert-butyl ether, MTBE), isobutyl acetate, or a mixture thereof.

In one embodiment, according to the present invention, the first crystallization solvent may be used in an amount sufficient to completely dissolve the compound of formula 1.

In one embodiment, according to the present invention, the organic acid may be contained in an excess molar ratio compared to the compound of formula 1 in the mixed solution. For example, the molar ratio of the compound of formula 1 and the organic acid in the mixed solution may be 1:1 to 1:2.

Dissolution of the crude compound of formula 1 in the first crystallization solvent may be carried out without or with stirring at room temperature, for example, 15 to 30°C, and specifically at a temperature of 23 to 28°C.

Next, crystals are obtained from the mixed solution in which the compound of formula 1 and the organic acid have been dissolved.

The crystals may be obtained, for example, by cooling the solution, by evaporating the solvent, by adding an antisolvent for supersaturation, or by using methods, such as slurry conversion, or the like.

In one embodiment, according to the present invention, the step of obtaining crystals may include additionally introducing a second crystallization solvent different from the first crystallization solvent to the mixed solution.

The yield or production stability of the obtained crystalline form IV may be improved by adding the second crystallization solvent to increase the production rate of crystallized particles, but the present invention is not limited thereto.

The second crystallization solvent may be an organic solvent of the above-described first crystallization solvent. However, preferably a solvent different from the solvent used as the first crystallization solvent may be selected and used.

In addition, in one embodiment, according to the present invention, the step of obtaining crystals may further include at least one of stirring the mixed solution and filtering the mixed solution, in any order.

The crystalline form IV as obtained above may have higher purity than a crude compound of formula 1, an amorphous compound of formula 1, other pharmaceutically acceptable salts of the compound of formula 1, or any crystalline forms of formula 1, and may be physically and chemically more stable. However, the effects of the present invention are not limited thereto.

In another aspect, the present invention provides a pharmaceutical composition comprising: (i) the crystalline form IV; and (ii) a pharmaceutically acceptable carrier.

The crystalline form IV, according to the present invention, exhibits excellent agonistic actions on melanocortin receptors, in particular, melanocortin-4 receptors (MC4R). Thus, the present invention can provide a pharmaceutical composition for agonizing melanocortin receptors, the composition containing the above-described crystalline form IV as an active ingredient. Specifically, the pharmaceutical composition may be a composition for agonizing the function of the melanocortin-4 receptor.

In addition, since the pharmaceutical composition can exhibit excellent effects of preventing or treating obesity, diabetes, inflammation, and erectile dysfunction, it may be a composition for preventing or treating obesity, diabetes, inflammation, or erectile dysfunction. However, the use of the present invention is not limited the diseases.

As used herein, "carrier" refers to a compound that facilitates the introduction of compounds into a cell or tissue.

When the crystalline form IV of the present invention is administered for clinical purposes, the total daily dose to be administered to a host in a single dose or in divided doses may be preferably in the range of 0.01 to 10 mg/kg body weight. However, the specific dose level for an individual patient may vary depending on the specific compound to be used, the patient's weight, sex, health status, diet, administration time of the drug, administration method, excretion rate, drug combination, the severity of the disease, or the like.

The crystalline form IV of the present invention may be administered by any route as desired. For example, the crystalline form IV of the present invention may be administered by injection or orally.

The pharmaceutical composition of the present invention may be in various oral dosage forms, such as tablets, pills, powders, capsules, granules, syrups, or emulsions, or parenteral dosage forms, such as injection preparations for intramuscular, intravenous, or subcutaneous administration.

Preparations for injection may be prepared, according to known techniques, using suitable dispersing agents, wetting agents, suspending agents, or excipients.

Excipients that can be used in the pharmaceutical preparation of the present invention include, but are not limited to, sweeteners, binders, solubilizers, solubilizing agents, wetting agents, emulsifiers, isotonic agents, adsorbents, disintegrants, antioxidants, preservatives, lubricants, fillers, fragrances, etc. For example, as excipients, lactose, dextrose, sucrose, mannitol, sorbitol, cellulose, glycine, silica, magnesium aluminum silicate, starch, gelatin, gum tragacanth, arginic acid, sodium alginate, methylcellulose, sodium carboxymethyl cellulose, water, ethanol, polyethylene glycol, polyvinyl pyrrolidone, sodium chloride, calcium chloride, orange essence, strawberry essence, vanilla flavor, etc. may be used.

When the pharmaceutical composition of the present invention is in an oral dosage form, examples of the carrier to be used may include, but are not limited to, cellulose, calcium silicate, corn starch, lactose, sucrose, dextrose, calcium phosphate, stearic acid, magnesium stearate, calcium stearate, gelatin, talc, etc.

When the pharmaceutical composition of the present invention is in an injectable preparation form, examples of the carrier may include, but are not limited to, water, saline, aqueous glucose solution, an aqueous sugar-like solution, alcohols, glycol, ethers, oils, fatty acids, fatty acid esters, glycerides, etc.

In another aspect, there is provided a crystalline form IV as described above for use in agonizing the functions of melanocortin receptors, in particular, melanocortin-4 receptors (MC4R).

In one embodiment, there is provided a crystalline form IV as described above for use in treating or preventing obesity, diabetes, inflammation, or erectile dysfunction.

In another aspect, there is provided a method for agonizing the function of melanocortin receptors, in particular, melanocortin-4 receptors (MC4R), the method comprising a step for administering to a subject the above-described crystalline form IV.

In another aspect, there is provided a method for treating obesity, diabetes, inflammation, or erectile dysfunction, the method comprising a step for administering to a subject the above-described crystalline form IV.

### ADVANTAGEOUS EFFECTS

The crystalline form IV, according to the present invention, exhibits excellent agonistic action on melanocortin receptors, in particular, melanocortin-4 receptors (MC4R), and thus can be usefully used for preventing or treating obesity, diabetes, inflammation, and erectile dysfunction.

The crystalline form IV, according to the present invention, exhibits an on-target effect on melanocortin-4 receptors, thereby exhibiting weight loss and diet reduction effects, without affecting anxiety and depression. In addition, it can be administered without any safety issues, such as side effects of human ether-a-go-go related gene (hERG) inhibition or mutagenesis.

In addition, the crystalline form IV, according to the present invention, has purity, yield, physical and chemical stability, which are more excellent than the crude compound of formula 1, the amorphous compound of formula 1, other pharmaceutically acceptable salts of the compound of formula 1, or any other crystalline forms of formula 1.

Specifically, the crystalline form IV may have superior solubility, storage stability, and production stability to the compound of formula 1, the amorphous compound of formula 1, other pharmaceutically acceptable salts of the compound of formula 1, or any other crystalline forms of formula 1.

### BRIEF DESCRIPTION OF THE DRAWINGS

FIG. 1 is a graph of the XRPD result of Example 1.
FIG. 2 is a graph of the TGA (top) and DSC (bottom) results of Example 1.
FIG. 3 is the HSM photograph of Example 1 (20x0.40 magnification).
FIG. 4 is the HSM photograph after drying Example 1 (20x0.40 magnification).
FIG. 5 is a graph of the XRPD result of Example 2.
FIG. 6 is a graph of the TGA (top) and DSC (bottom) results of Example 2.
FIG. 7 is the HSM photograph after drying Example 2 (20x0.40 magnification).
FIG. 8 is the HSM photograph after drying Example 2 (20x0.40 magnification).
FIG. 9 is a graph of the XRPD result of Example 3.
FIG. 10 is a graph of the TGA (top) and DSC (bottom) results of Example 3.
FIG. 11 is a spectrum of the ¹H NMR result of Example 3.

### MODE FOR CARRYING OUT THE INVENTION

Hereinafter, the present invention will be described in more detail through Preparation Examples and Examples. However, these Examples are merely illustrative of the present invention, and the scope of the present invention is not limited thereto.

### Preparation Example 1: Preparation of methyl (2S,4S)-4-(N-((1s,4R)-4-methylcyclohexyl)isobutyramido)pyrrolidine-2-carboxylate hydrochloride

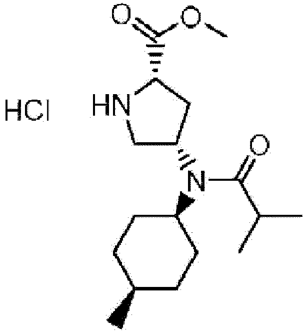

The title compound was obtained through the following steps A, B, C, D, and E.

### Step A: Preparation of 1-(tert-butyl) 2-methyl (2S,4S)-4-azidopyrrolidine-1,2-dicarboxylate

1-(tert-butyl) 2-methyl (2S,4R)-4-((methylsulfonyl)oxy)pyrrolidine-1,2-dicarboxylate (48.5 g, 150 mmol) was dissolved in N,N'-dimethylformamide (250 ml) under nitrogen, and sodium azide (19.5 g, 300 ml) was added. After stirring at 80°C for 16 hours, the reaction solvent was concentrated under reduced pressure, water was added, and extraction was performed twice with ethyl acetate. The organic layer was washed with an aqueous sodium chloride solution and water, dried over anhydrous magnesium sulfate, and filtered. The filtrate was concentrated under reduced pressure to obtain crude 1-(tert-butyl) 2-methyl (2S,4S)-4-azidopyrrolidine-1,2-dicarboxylate (39.59 g, 98%), which was used in the next step without purification.
MS [M+H] = 271 (M+1)
¹H NMR (400 MHz, CD3OD) δ 4.43-4.37 (m, 1H), 4.35-4.27 (br, 1H), 3.77 (s, 1.8H), 3.76 (s, 1.2H), 3.73-3.66 (m, 1H), 3.44-3.38 (m, 1H), 2.63-2.49 (m, 1H), 2.19-2.11 (m, 1H), 1.50 (s, 4.5H), 1.44 (s, 4.5H)

### Step B: Preparation of 1-(tert-butyl) 2-methyl (2S,4S)-4-aminopyrrolidine-1,2-dicarboxylate

1-(tert-butyl) 2-methyl (2S,4S)-4-azidopyrrolidine-1,2-dicarboxylate (24.59 g, 91.0 mmol) obtained in step A above was dissolved in tetrahydrofuran (180 ml), and 1 M trimethylphosphine tetrahydro solution (109.2 ml, 109.2 mmol) was slowly added at 0°C. The mixture was stirred at the same temperature for 1 hour and then at room temperature for 3 hours. After the reaction solvent was concentrated under reduced pressure, dichloromethane (100 ml) and water (150 ml) were added, and the mixture was stirred for about 30 minutes. The layers were separated and were extracted once more with dichloromethane, and the organic layer was dried over anhydrous magnesium sulfate and was filtered. The filtrate was concentrated under reduced pressure to obtain crude 1-(tert-butyl) 2-methyl (2S,4S)-4-aminopyrrolidine-1,2-dicarboxylate (20.62 g, 93%), which was used in the next step without purification.
MS [M+H] = 245 (M+1)
^{1H} NMR (400 MHz, CD3OD) δ 4.27 (m, 1H), 3.77 (s, 1.8H), 3.76 (s,1.2H), 3.75-3.67 (m, 1H), 3.50-3.42 (m, 1H), 3.22-3.17 (m, 1H), 2.58-2.47 (m,1H), 1.82-1.71 (m, 1H), 1.48 (s, 4.5H), 1.42 (s, 4.5H)

### Step C: Preparation of 1-(tert-butyl) 2-methyl (2S,4S)-4-(((1s,4R)-4-methylcyclohexyl)amino)pyrrolidine-1,2-dicarboxylate

1-(tert-butyl) 2-methyl (2S,4S)-4-aminopyrrolidine-1,2-dicarboxylate (20.62 g, 84.4 mmol) obtained in step B above was dissolved in dichloroethane (150 ml), and 4-methylcyclohexanone (9.5 ml, 101.3 mmol) was added. Sodium triacetoxyborohydride (26.8 g, 126.6 mmol) was added at 0°C, and the mixture was stirred at room temperature for 16 hours. The reaction solvent was concentrated under reduced pressure, water was added, and extraction was performed twice with ethyl acetate. The organic layer was washed with an aqueous sodium chloride solution, dried over anhydrous magnesium sulfate, and filtered. The filtrate was concentrated under reduced pressure and purified by column chromatography to obtain 1-(tert-butyl) 2-methyl (2S,4S)-4-(((1s,4R)-4-methylcyclohexyl)amino)pyrrolidine-1,2-dicarboxylate (22.9 g, 80%) .
MS [M+H] = 341 (M+1)
^{1H} NMR (400 MHz, CD3OD) δ 4.26 (m, 1H), 3.76 (s, 1.8H), 3.75 (s, 1.2H), 3.78-3.71 (m, 1H), 3.49-3.40 (m, 1H), 3.22-3.16 (m, 1H), 2.69-2.60(br, 1H), 2.58-2.46 (m, 1H), 1.87-1.77 (m, 1H), 1.73-1.63 (m, 1H), 1.62-1.35(m, 8H), 1.48 (s, 4.5H), 1.42 (s, 4.5H), 0.96 (d, 3H)

### Step D: Preparation of 1-(tert-butyl) 2-methyl (2S,4S)-4-(N-((1s,4R)-4-methylcyclohexyl)isobutyramido)pyrrolidine-1,2-dicarboxylate

1-(tert-butyl) 2-methyl (2S,4S)-4-(((1s,4R)-4-methylcyclohexyl)amino)pyrrolidine-1,2-dicarboxylate obtained in step C above (37.29 g, 109.5 mmol) was dissolved in dichloromethane (500 ml), triethyl amine (61.1 ml, 438.1 mmol) was added, and then isobutyryl chloride (11.7 ml, 219 mmol) was slowly added at 0°C. After the mixture was stirred at room temperature for 16 hours, the reaction solvent was concentrated under reduced pressure, an aqueous sodium hydrogen carbonate solution was added, and extraction was performed twice with ethyl acetate. The organic layer was washed with an aqueous sodium chloride solution and water, dried over anhydrous magnesium sulfate, and filtered. The filtrate was concentrated under reduced pressure, and purified by column chromatography to obtain 1-(tert-butyl) 2-methyl (2S,4S)-4-(N-((1s,4R)-4-methylcyclohexyl)isobutyramido)pyrrolidine-1,2-dicarboxylate (38.79 g, 86%).
MS [M+H] = 411 (M+1)
^{1H}NMR (400 MHz, CD3OD) δ 4.27 (m, 1H), 3.76 (s, 1.8H), 3.75 (s, 1.2H), 3.78-3.72 (m, 1H), 3.50-3.41 (m, 1H), 3.33-3.14 (m, 1H), 2.69-2.60 (m, 2H), 2.57-2.43 (m, 1H), 1.87-1.79 (m, 1H), 1.70-1.61 (m, 1H), 1.60-1.32 (m, 8H), 1.47 (s, 4.5H), 1.41 (s, 4.5H), 1.10 (dd, 6H), 0.99 (d, 3H)

### Step E: Preparation of methyl (2S,4S)-4-(N-((1s,4R)-4-methylcyclohexyl)isobutyramido)pyrrolidine-2-carboxylate hydrochloride

1-(tert-butyl) 2-methyl (2S,4S)-4-(N-((1s,4R)-4-methylcyclohexyl)isobutyramido)pyrrolidine-1,2-dicarboxylate (34.0 g, 82.8 mmol) obtained in step D above was dissolved in dichloromethane (200 ml), and a solution of 4 N hydrochloric acid in 1,4-dioxane solution (82.8 ml, 331.3 mmol) was added at 0°C. After the mixture was stirred at room temperature for 6 hours, the reaction solvent was concentrated under reduced pressure to obtain crude (28.7 g, 99%), which was used in the next step without purification.
MS[M+H] = 311 (M+1)

### Preparation Example 2: Preparation of (3S,4R)-1-(tert-butyl)-4-(4-chlorophenyl)pyrrolidine-3-carboxylic acid

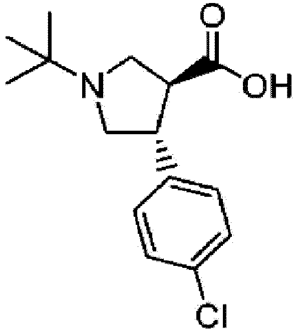

The title compound was obtained according to the method described in international patent publication no. WO 2004/092126.
MS[ M+H] = 282 (M+1)
^{1H} NMR (400 MHz, CD3OD) δ 7.43-7.33 (m, 4H), 3.90-3.69 (m, 3H), 3.59 (dd, J = 11.2, 10.0 Hz, 1H), 3.29 (dd, J = 11.2, 11.2 Hz, 1H), 3.18-3.09 (m, 1H), 1.44 (s, 9H)

### Preparation Example 3: Preparation of N-((3S,5S)-1-((3S,4R)-1-(tert-butyl)-4-(4-chlorophenyl)

### pyrrolidine-3-carbonyl)-5-(morpholine-4-carbonyl)pyrrolidin-3-yl)-N-((1s,4R)-4-methylcyclohexyl)isobutyramide (MC70)

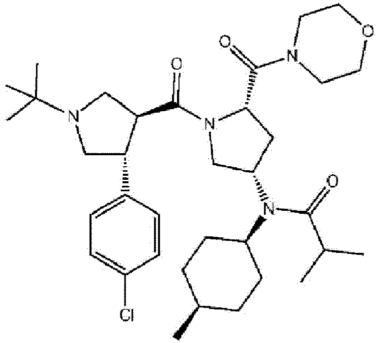

The title compound was obtained through the following steps A, B, and C.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate

Methyl (2S,4S)-4-(N-((1s,4R)-4-methylcyclohexyl)isobutyramido)pyrrolidine-2-carboxylate hydrochloride (28.7 g, 82.73 mmol) obtained in Preparation Example 1, (3S,4R)-1-(tert-butyl)-4-(4-chlorophenyl)pyrrolidine-3-carboxylic acid (24.5 g, 86.87 mmol) obtained in Preparation Example 2, 1-(3-dimethylaminopropyl)-3-ethylcarbodiimide hydrochloride (22.2 g, 115.83 mmol), and 1-hydroxybenzotriazole hydrate (15.7 g, 115.83 mmol) were dissolved in N,N'-dimethylformamide (400 ml), and N,N'-diisopropylethylamine (72.0 ml, 413.66 mmol) was added slowly. The mixture was stirred at room temperature for 16 hours, the reaction solvent was concentrated under reduced pressure, 0.5 N sodium hydroxide aqueous solution was added, and then, extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (41.19 g, 87%).
MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid

Methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (39.4 g, 68.62 mmol) obtained in step A above was dissolved in methanol (450 ml), and then, 6 N sodium hydroxide aqueous solution (57.2 ml, 343.09 mmol) was added. The mixture was stirred at room temperature for 16 hours, the pH was adjusted to about 5 with 6 N aqueous hydrochloric acid solution, and then the reaction solution was concentrated under reduced pressure. The concentrate was dissolved in dichloromethane, and then the insoluble solid was filtered through a paper filter. The filtrate was concentrated under reduced pressure to obtain the crude title compound (38.4 g, 99%), which was used in the next step without purification.
MS [M+H] = 561 (M+1)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

(2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid (38.4 g, 68.60 mmol) obtained in step B above, 1-(3-dimethylaminopropyl)-3-ethylcarbodiimide hydrochloride (18.4 g, 96.04 mmol), and 1-hydroxybenzotriazole hydrate (13.0 g, 96.04 mmol) were dissolved in N,N'-dimethylformamide (200 ml), and then morpholine (5.9 ml, 68.80 mmol) and N,N'-diisopropylethylamine (59.7 ml, 343.02 mmol) were sequentially and slowly added. The mixture was stirred at room temperature for 16 hours, the reaction solution was concentrated under reduced pressure, 0.5 N sodium hydroxide aqueous solution was added, and then extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide (37.05 g, 86%, **MC70**).
MS [M+H] = 630 (M+1)

### Example 1: Preparation of crystalline form of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide lactate

The compound (MC70) prepared in Preparation Example 3 and 85% lactic acid aqueous solution in a molar ratio of 1:1 were added to ethanol. Then, stirring was carried out at room temperature for 1 day, and the solvent was evaporated with the lid open at room temperature. Heptane was added dropwise, the mixture was further stirred at room temperature for 1 day to obtain the title compound (a crystalline form of lactate of MC70).

### Example 2: Preparation of crystalline form of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide benzene sulfonate

### [Example 2-1]

The compound (MC70) prepared in Preparation Example 3 and benzene sulfonic acid in a molar ratio of 1:1 were added to ethanol, and the mixture was stirred at room temperature for 1 day. Ethanol was evaporated so that the mixture was gelated, heptane was added, and the mixture was stirred at room temperature for 4 days to obtain the title compound (a crystalline form of benzene sulfonate of MC70) as a sticky solid in solution.

### [Example 2-2]

The compound (MC70) prepared in Preparation Example 3 and benzene sulfonic acid in a molar ratio of 1:1 were added to toluene, and the mixture was stirred at room temperature for 4 days. Toluene was evaporated so that the mixture was gelated, MTBE was added, and the mixture was stirred at room temperature for 4 days to maintain the gel state, but MTBE was evaporated, cyclohexane was then added, and the mixture was stirred at 50°C for 2 days to obtain the title compound (a crystalline form of benzene sulfonate of MC70) in a solid state.

### [Example 2-3]

The compound (MC70) prepared in Preparation Example 3 and benzene sulfonic acid in a molar ratio of 1:1 were added to MTBE, and the mixture was stirred at room temperature in a suspension state for 1 hour. The mixture was further stirred at 40°C for 4 days, MTBE was evaporated, cyclohexane was added, and the mixture was stirred at 50°C for 2 days. A mixed state of the crystalline form and the amorphous form in the suspension was observed. The mixture was stirred at 60°C for 6 days, toluene was added, and the mixture was stirred at 60°C for 6 days to finally obtain the title compound (a crystalline form of benzene sulfonate of MC70) in a mixed state of the crystalline form and the amorphous form.

### Example 3: Preparation of crystalline form of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide toluene sulfonate

### [Example 3-1]

Toluene sulfonate (p-toluene sulfonate) was added in an equivalent ratio of 1:1 to a solution of the compound (MC70) prepared in Preparation Example 3 in isobutyl acetate, followed by stirring at room temperature for 1 day. A portion of the solvent was evaporated with the lid open at room temperature, and then, cyclohexane was added, and the mixture was stirred at room temperature for 1 day. Finally, MTBE was added, and the mixture was stirred at room temperature for 4 days, and then at 40°C for 1 day to obtain the title compound (a crystalline form of toluene sulfonate of MC70) .

### [Example 3-2]

Toluene sulfonate (p-toluene sulfonate) was added in an equivalent ratio of 1:1 to a solution of the compound (MC70) prepared in Preparation Example 3 in XX mL of ethanol, followed by stirring at room temperature for 6 hours. The lid was open at room temperature, and nitrogen gas was injected to evaporate the solution. Then, XX mL of MTBE was added, and the mixture was stirred at 40°C for 1 day, and then at 50°C for 1 day to obtain the title compound (a crystalline form of toluene sulfonate of MC70) .

For the compounds of Examples 1 to 3, XRPD (FIGs. 1, 5, and 9), TGA/DCS (FIGs. 2, 6, and 10), HSM (FIGs. 3, 7, and 8), and 1H NMR (FIG. 11) were analyzed, and graphs of the results are shown in FIGs. 1 to 11.

### Comparative Example 1: Preparation of crystalline form of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide toluene adipate

The compound (MC70) prepared in Preparation Example 3 was added to ethanol, and adipic acid was mixed in an equivalent ratio of 1:1 to prepare a mixed solution. The mixed solution was stirred to be gelated, and then heptane and MTBE were sequentially added, but no crystals formed.

### Comparative Example 2: Preparation of crystalline form of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide toluene adipate

The compound (MC70) prepared in Preparation Example 3 was added to isopropyl alcohol, and adipic acid was mixed in an equivalent ratio of 1:8 to prepare a mixed solution. The mixed solution was stirred to be gelated, and then heptane was added, but no crystals formed.

### Experimental Example 1. XRPD assessment

Powder XRPD diffraction patterns were obtained with Panalytical Xpert Pro MPD diffractometer using an incident beam of Cu radiation. About 20 to 30 mg of the sample was compressed in a glass sample holder so that the sample had a flat surface, the generator of the apparatus was set to 45 kV (acceleration voltage) and 40 mA (filament emission), and then, the measurement was carried out in a reflection mode (notspin). Bragg angles (2θ) in a range of 4 to 40° were measured with the conditions of step size of 0.026° and time per step of 51 seconds.

As can be seen from the spectrum shown in FIG. 1, it was confirmed that the crystalline form IV of the lactate according to Example 1 was a crystalline substance. The specific values of the XRPD are shown in Table 1 below.

**[Table 1]**

| No. | 2θ | **Relative intensity**(l/l₀) |
|---|---|---|
| 1 | 3.79 | 158.43 |
| 2 | 7.6252 | 3524.59 |
| 3 | 9.3554 | 9494.31 |
| 4 | 9.5981 | 4389.53 |
| 5 | 9.914 | 1032.8 |
| 6 | 10.3 | 31.35 |
| 7 | 10.9428 | 2175.37 |
| 8 | 11.0934 | 1230.94 |
| 9 | 11.7645 | 2056.27 |
| 10 | 12.591 | 4210.17 |
| 11 | 13.3233 | 1284.89 |
| 12 | 13.527 | 837 |
| 13 | 14.0794 | 3181.72 |
| 14 | 14.9866 | 3322.28 |
| 15 | 15.259 | 3296.87 |
| 16 | 16.268 | 755.1 |
| 17 | 16.526 | 566.07 |
| 18 | 16.7638 | 5751.62 |
| 19 | 17.1836 | 7358.63 |
| 20 | 17.544 | 577.03 |
| 21 | 17.6 | 58.6 |
| 22 | 17.931 | 919.1 |
| 23 | 18.86 | 3311.42 |
| 24 | 18.89 | 3019.25 |
| 25 | 18.996 | 4517.05 |
| 26 | 19.2114 | 3730.41 |
| 27 | 19.368 | 3712.44 |
| 28 | 20.1323 | 6460..48 |
| 29 | 20.3806 | 5810.33 |
| 30 | 20.626 | 4795.85 |
| 31 | 20.718 | 4044.66 |
| 32 | 20.931 | 863.96 |
| 33 | 21.2828 | 2942.18 |
| 34 | 21.834 | 1519.33 |
| 35 | 21.941 | 2213.05 |
| 36 | 22.1357 | 3381.94 |
| 37 | 22.3272 | 4096.68 |
| 38 | 22.9116 | 1835.3 |
| 39 | 23.294 | 684.26 |
| 40 | 23.6289 | 1574.26 |
| 41 | 24.22 | 217.88 |
| 42 | 24.987 | 1470.72 |
| 43 | 25.338 | 1031.04 |
| 44 | 25.841 | 230.62 |
| 45 | 26.838 | 885.2 |
| 46 | 27.45 | 294.53 |
| 47 | 28.203 | 427.79 |
| 48 | 28.471 | 1699.81 |
| 49 | 28.892 | 821.1 |
| 50 | 29.078 | 1242.11 |
| 51 | 29.527 | 214.51 |
| 52 | 29.863 | 778 |
| 53 | 30.021 | 955.15 |
| 54 | 30.292 | 714.56 |
| 55 | 30.898 | 971.27 |
| 56 | 31.108 | 2167.04 |
| 57 | 31.621 | 270.46 |
| 58 | 31.929 | 389.54 |
| 59 | 32.585 | 348.99 |
| 60 | 32.958 | 428.91 |
| 61 | 33.349 | 356.54 |
| 62 | 33.933 | 157.07 |
| 63 | 34.491 | 114.08 |
| 64 | 35.621 | 115.64 |
| 65 | 36.384 | 328.76 |
| 66 | 37.567 | 228.31 |
| 67 | 38.477 | 239.81 |
| 68 | 39.063 | 294.49 |

As can be seen from the spectrum shown in FIG. 5, it was confirmed that the crystalline form IV of the benzene sulfonate according to Example 2 was a crystalline substance. The specific values of the XRPD are shown in Table 2 below.

**[Table 2]**

| No. | 2θ | **Relative intensity** (l/l₀) |
|---|---|---|
| 1 | 8.4669 | 8241.67 |
| 2 | 10.715 | 896.22 |
| 3 | 10.859 | 1873.64 |
| 4 | 11.807 | 260.1 |
| 5 | 11.985 | 922.64 |
| 6 | 12.213 | 900.12 |
| 7 | 13.4746 | 4789.65 |
| 8 | 15.084 | 2089.49 |
| 9 | 15.186 | 2911.82 |
| 10 | 16.804 | 1392.78 |
| 11 | 17.251 | 1687.45 |
| 12 | 17.4479 | 4305.61 |
| 13 | 17.8609 | 5627.11 |
| 14 | 18.07 | 515.57 |
| 15 | 18.7707 | 4054.63 |
| 16 | 19.055 | 444.74 |
| 17 | 19.22 | 667.1 |
| 18 | 20.261 | 1560.65 |
| 19 | 20.441 | 1151.56 |
| 20 | 20.853 | 730.99 |
| 21 | 21-193 | 840.61 |
| 22 | 21.512 | 902.45 |
| 23 | 21.811 | 2411.4 |
| 24 | 22.004 | 1291.31 |
| 25 | 22.662 | 1449.68 |
| 26 | 22.993 | 393.95 |
| 27 | 23.504 | 204.68 |
| 28 | 23.74 | 122.07 |
| 29 | 24.305 | 195.82 |
| 30 | 24.4 | 158.34 |
| 31 | 24.55 | 80.35 |
| 32 | 24.968 | 997.63 |
| 33 | 25.358 | 1542.86 |
| 34 | 25.743 | 561 .04 |
| 35 | 26.214 | 496.13 |
| 36 | 26.79 | 297.84 |
| 37 | 27.137 | 625.39 |
| 38 | 27.587 | 206.68 |
| 39 | 28.509 | 367.2 |
| 40 | 29.367 | 292.12 |
| 41 | 29.832 | 239.82 |
| 42 | 30.473 | 703.25 |
| 43 | 31.006 | 142.83 |
| 44 | 31.382 | 153.55 |
| 45 | 32.218 | 130.15 |
| 46 | 33.309 | 128.18 |
| 47 | 33.719 | 162.52 |
| 48 | 34.84 | 94.21 |
| 49 | 36.535 | 142.64 |
| 50 | 37.45 | 44.68 |
| 51 | 38.59 | 95.56 |
| 52 | 39.5 | 85.63 |

As can be seen from the spectrum shown in FIG. 9, it was confirmed that the crystalline form IV of the toluene sulfonate according to Example 3 was a crystalline substance. The specific values of the XRPD are shown in Table 3 below.

**[Table 3]**

| No. | 2θ | **Relative intensity** (l/l₀) |
|---|---|---|
| 1 | 4.2678 | 4162.11 |
| 2 | 4.9412 | 1588.89 |
| 3 | 6.5584 | 5034.31 |
| 4 | 7.4521 | 1967.4 |
| 5 | 7.745 | 340.4 |
| 6 | 8.68 | 669.96 |
| 7 | 8.8659 | 5605.49 |
| 8 | 9.8591 | 2516.94 |
| 9 | 10.469 | 2171.38 |
| 10 | 10.856 | 108.77 |
| 11 | 11.6 | 1266.61 |
| 12 | 12.401 | 1353.75 |
| 13 | 13.176 | 1470.18 |
| 14 | 13.595 | 1712.93 |
| 15 | 14.1.6 | 841.81 |
| 16 | 14.721 | 2390.5 |
| 17 | 14.931 | 1328.17 |
| 18 | 15.156 | 2353.27 |
| 19 | 15.351 | 1483.73 |
| 20 | 15.563 | 2169.58 |
| 21 | 15.739 | 3063.61 |
| 22 | 16.143 | 3222.78 |
| 23 | 16.39 | 1404.68 |
| 24 | 16.5 | 1026.65 |
| 25 | 16.53 | 1766.54 |
| 26 | 16.72 | 2429.44 |
| 27 | 16.905 | 3753.15 |
| 28 | 17.093 | 6144.96 |
| 29 | 17.274 | 5869.08 |
| 30 | 17.52 | 2276.53 |
| 31 | 17.811 | 2887.32 |
| 32 | 17.998 | 3493.81 |
| 33 | 18.333 | 3273.32 |
| 34 | 18.86 | 1254.15 |
| 35 | 18.92 | 2096.74 |
| 36 | 19.023 | 1234.05 |
| 37 | 19.341 | 1343.62 |
| 38 | 19.522 | 1386.4 |
| 39 | 19.668 | 3443.77 |
| 40 | 19.858 | 978.24 |
| 41 | 20.3037 | 3616.31 |
| 42 | 21.2264 | 4065.85 |
| 43 | 21.41 | 1198.6 |
| 44 | 21.831 | 384.75 |
| 45 | 22.1 | 687.85 |
| 46 | 22.308 | 1632.61 |
| 47 | 22.632 | 459.83 |
| 48 | 23.073 | 1068.49 |
| 49 | 23.438 | 1366.13 |
| 50 | 23.834 | 1603.53 |
| 51 | 23.966 | 3574.42 |
| 52 | 24.25 | 438.28 |
| 53 | 25.017 | 799.79 |
| 54 | 25.343 | 383.07 |
| 55 | 25.868 | 753.83 |
| 56 | 26.921 | 568.29 |
| 57 | 27.26 | 387.65 |
| 58 | 28.407 | 852.27 |
| 59 | 29.723 | 311.33 |
| 60 | 30.056 | 416.61 |
| 61 | 31.094 | 412.78 |
| 62 | 31.653 | 565.69 |
| 63 | 32.346 | 438.45 |
| 64 | 33.735 | 176.2 |
| 65 | 34.106 | 213.66 |
| 66 | 36.14 | 138.64 |
| 67 | 37.397 | 206.52 |
| 68 | 38.13 | 163.35 |
| 69 | 39.2 | 311.81 |

### Experimental Example 2. Thermogravimetric analysis (TGA) and differential scanning calorimetry (DSC)

TGA and DSC combo analysis was performed with Mettler-Toledo TGA/DSC 3+ analyzer. The sample was placed in an open aluminum pan, the pan was sealed, the lid was pierced, and then the pan was inserted into the TG furnace. Measurements were made by heating from 25°C to a maximum of 350°C at a rate of 10 K/min under nitrogen.

As can be seen in FIG. 2, according to the TGA analysis result, the crystalline form IV of the lactate according to Example 1 had a weight loss of about 1.0 wt% in a temperature range of about 38°C to 100°C and was decomposed beyond about 274°C.

In addition, according to the DSC analysis result, for the crystalline form IV of the lactate according to Example 1, endothermic peaks were observed at about 82°C and 103°C, and an exothermic peak was observed at about 116°C (peak).

As can be seen in FIG. 6, according to the TGA analysis result, the crystalline form IV of the benzene sulfonate according to Example 2 had a weight loss of about 5.4 wt% in a temperature range of about 39°C to 150°C and was decomposed beyond about 266°C.

In addition, according to the DSC analysis result, for the crystalline form IV of the benzene sulfonate according to Example 2, broad endothermic peaks were observed at about 57°C and 93°C.

As can be seen in FIG. 10, according to the TGA analysis result, the crystalline form IV of the toluene sulfonate according to Example 3 had a weight loss of about 5.3 wt% in a temperature range of about 46°C to 135°C and was decomposed beyond about 264°C.

In addition, according to the DSC analysis result, for the crystalline form IV of the toluene sulfonate according to Example 3, an endothermic peak was observed at about 104°C, and an exothermic peak was observed at about 138°C (peak).

### Experimental Example 3. Hot Stage Microscopy (HSM)

HSM was measured with Leica DM LP microscope using Linkman hot stage (model FTIR 600) with TMS 93 controller. Samples were observed with a lambda plate with crossed polarizers at either 10x0.22 or 20x0.40 magnification. Samples were placed on a coverslip, and another coverslip was placed over the sample. Then, the sample was visually observed as the stage was heated. In order to investigate outgassing, in some cases, a drop of mineral oil may be added to the sample. Images were captured using SPO Insight color digital camera with SPOT software v.4.5.9.

As can be seen in FIGs. 3 and 4, according to the HSM analysis result, it was confirmed that the crystalline form IV of the lactate according to Example 1 melted at about 84.2 to 112.7°C.

As can be seen in FIGs. 7 and 8, according to the TGA analysis result, according to the HSM analysis result, it was confirmed the crystalline form IV of the benzene sulfonate according to Example 2 melted at about 122°C to 157°C.

### Experimental Example 4. Solubility assessment

Solubility was measured based on the final volume of water added to a sample when it was visually confirmed that the sample was completely dissolved by adding water to the sample.

According to the assessment result, the solubility of the crystalline form IV of the lactate according to Example 1 was more than 24 mg/mL. The solubility of the crystalline form IV of the benzene sulfonate according to Example 2 was more than 1 mg/mL. In addition, the solubility of the crystalline form IV of the toluene sulfonate according to Example 3 was more than 10 mg/mL.

As described above, it was confirmed that the crystalline forms according to Examples 1 to 3 had excellent solubility in water.

## Claims

1. A crystalline form IV of an organic acid salt of a compound of the following formula 1,
wherein the X-ray powder diffraction (XRPD) pattern has characteristic peaks with diffraction angles (2θ values) described in i), ii), or iii):
i) 5 or more characteristic peaks selected from among 7.63±0.2°, 9.36±0.2°, 9.60±0.2°, 12.59±0.2°, 14.0±0.2°, 14.99±0.2°, 15.26±0.2°, 16.76±0.2°, 17.8±0.2°, 18.86±0.2°, 18.89±0.2°, 19.00±0.2°, 19.21±0.2°, 19.37±0.2°, 20.13±0.2°, 20.38±0.2°, 20.63±0.2°, 20.72±0.2°, 22.14±0.2°, and 22.33±0.2°;
ii) 5 or more characteristic peaks selected from among 8.47±0.2°, 10.86±0.2°, 11.99±0.2°, 12.21±0.2°, 13.47±0.2°, 15.08±0.2°, 15.19±0.2°, 16.80±0.2°, 17.25±0.2°, 17.45±0.2°, 17.86±0.2°, 18.77±0.2°, 20.26±0.2°, 20.44±0.2°, 21.51±0.2°, 21.81±0.2°, 22.00±0.2°, 22.66±0.2°, 24.97±0.2°, and 25.36±0.2°;
iii) 5 or more characteristic peaks selected from among 4.27±0.2°, 6.56±0.2°, 8.87±0.2°, 9.86±0.2°, 14.7210.2°, 15.16±0.2°, 15.74±0.2°, 16.14±0.2°, 16.72±0.2°, 16.91±0.2°, 17.09±0.2°, 17.27±0.2°, 17.52±0.2°, 17.81±0.2°, 18.0010.2°, 18.33±0.2°, 19.67±0.2°, 20.30±0.2°, 21.23±0.2°, and 23.97±0.2°, 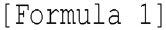 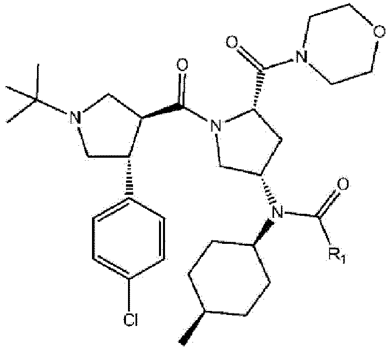
wherein
R₁ is a C₂-C₅ alkyl.

2. The crystalline form IV of claim 1, wherein R₁ is a C₂-C₄ alkyl.

3. The crystalline form IV of claim 2, wherein the compound of the formula 1 is *N*-((3S,5S)-1-((3S,4R)-1-(*tert*-butyl)-4-(4-chlorophenyl)pyrrolidine-3-carbonyl)-5-(morpholine-4-carbonyl)pyrrolidin-3-yl)-*N*-((1s,4R)-4-methylcyclohexyl)isobutyramide.

4. The crystalline form IV of claim 1, wherein the organic acid salt is selected from the group consisting of lactate, benzene sulfonate, and toluene sulfonate.

5. The crystalline form IV of claim 4, wherein the crystalline form IV is a crystalline form of a solvate with methyl tertiary-butyl ether of toluene sulfonate of the compound of the formula 1.

6. A method for preparing the crystalline form IV described in any one of claims 1 to 5, the method comprising the steps of:
preparing a mixed solution in which the compound of the formula 1 and an organic acid are mixed in a first crystallization solvent; and
obtaining crystals from the mixed solution.

7. The method for preparing the crystalline form IV of claim 6, wherein the organic acid is selected from the group consisting of lactic acid, benzene sulfonic acid, and toluene sulfonic acid.

8. The method for preparing the crystalline form IV of claim 6, wherein a molar ratio of the compound of the formula 1 and the organic acid in the mixed solution is 1:1 to 1:2.

9. The method for preparing the crystalline form IV of claim 6, wherein the step of obtaining crystals includes additionally introducing a second crystallization solvent different from the first crystallization solvent to the mixed solution.

10. The method for preparing the crystalline form IV of claim 6, wherein the step of obtaining crystals includes at least one of cooling the mixed solution, stirring the mixed solution, and filtering the mixed solution.

11. The method for preparing the crystalline form IV of claim 10, wherein the step of obtaining crystals includes stirring the mixed solution at a temperature of 20°C to 80°C.

12. A pharmaceutical composition comprising the crystalline form IV according to any one of claims 1-5 and a pharmaceutically acceptable carrier.

13. A pharmaceutical composition for agonizing the function of a melanocortin-4 receptor, the composition comprising the crystalline form IV according to any one of claims 1-5 and a pharmaceutically acceptable carrier.

14. The pharmaceutical composition of claim 13, which is for preventing or treating obesity, diabetes, inflammation, or erectile dysfunction.
